# EUROPEAN PATENT SPECIFICATION

(11) **EP 1 194 186 B1**
(45) Date of publication and mention of the grant of the patent: **02.05.2003**
(21) Application number: 00944233.6
(22) Date of filing: 03.07.2000
(51) Int. Cl.: A61P 9/00, A61K 35/78

(54) **CARDIOACTIVE COMPOSITION COMPRISING L-CARNITINE AND ITS DERIVATIVES AND i CRATAEGUS /i EXTRACTS**
KARDIOAKTIVE ZUSAMMENSETZUNG ENTHALTEND L-CARNITIN UND SEINE DERIVATE UND CRATAEGUS EXTRAKTE
COMPOSITION AGISSANT SUR LE COEUR RENFERMANT DE LA L-CARNITINE, DES DERIVES DE CELLE-CI, ET DES EXTRAITS DE i CRATAEGUS /i

(30) Priority: 09.07.1999 IT RM990436
(43) Date of publication of application: 10.04.2002
(73) Proprietor: SIGMA-TAU Industrie Farmaceutiche Riunite S.p.A., 00144 Roma (IT)
(72) Inventor: CAVAZZA, Claudio, I-00186 Roma (IT)
(74) Representative: Cavattoni, Fabio
(86) International application number: IT0000273
(87) International publication number: WO01003683

(56) References cited:
- US-A- 5 948 443
- US-A- 5 976 568
- B.S.KENDLER: "Recent nutritional approaches to the prevention and therapy of cardiovascular disease" PROGRESS IN CARDIOVASCULAR NURSING, vol. 12, no. 3, 1997, pages 3-23, XP000978849

## Description

The present invention relates to a composition comprising a combination of L-carnitine and its derivatives and *Crataegus* extracts. This combination presents a potent and surprising synergistic effect in stimulating myocardial activity and in regulating cardiac rhythm and coronary flow.

The composition can take the form and perform the activity of a dietary supplement or of an actual medicine, depending upon the support orpreventive or strictly therapeutic action which the composition is intended to exert in relation to the particular individuals for whom it is to be used in.

The favourable effects of both L-carnitine and its derivatives and of *Crataegus* extracts have long been known. However, the synergistic and mutually enhancing action which the combination of these compounds according to the invention is capable of exerting on cardiac activity has never been previously disclosed.

The favourable action exerted by the "carnitines" (a term which collectively refers to L-carnitine and the alkanoyl L-carnitines wherein the alkanoyl group has 2-5 carbon atoms) on both energy metabolism in general and myocardial metabolism in particular, is well known. Both L-carnitine, acetyl and propionyl L-carnitine are, in fact, therapeutically effective in myocardial insufficiency and in coronary disorders, as well as in peripheral vascular dysfunctions. Equally proven is the efficacy of these products in cardiac rhythm disorders. The mechanism of action of the carnitines underlying these effects accounts both for the better exploitation of energy resources through the processes of β-oxidation of fatty acids and glucose utilisation and the reduction of free radicals by inhibition of tissue oxidative processes.

Less well known is the cardioactive effect exerted by *Crataegus* extracts. Unlike the extracts of various plants, such as the lily of the valley (*Convallaria*), squill (*Scilla*), oleander (*Nerium oleander*) or pheasant's eye (*Adonis vernalis*), which mainly comprise cardenolides and act on the same receptors as digitalis, *Crataegus* extracts do not act on these receptors and possess a more complex action related to the presence in them of high concentrations of flavonoids and procyanidins. Among the flavonoids present, those identified and characterised are the O-heterosides of quercetol, such as hyperoside, spiraeoside, rutoside and crataeoside, and the flavonic C-heterosides derived from apigenin such as vitexin, rhamnylvitexin and isovitexin, of from luteolin, such as orientin, or the C-heterosides of apigenin, such as vicenin and iso- and neoshaftoside. Among the procyanidins, those identified are the flavan oligomers, particularly dimers and higher oligomers of epicatechol.

It has been proved by means of pharmacological tests that total extracts of *Crataegus* increase the cardiac contractile force without increasing oxygen consumption, increase coronary flow, reduce atrioventricular conduction time and, unlike digitalis compounds such as adrenaline and amrinone, prolong the myocardial refractory period.

In clinical practice, these effects mean that *Crataegus* extracts can advantageously be used particularly in subjects with chronic cardiac insufficiency, tachycardia or cardiac arrhythmia, hypertension, fatigue and poor resistance to effort.

It has now surprisingly been found that a composition containing a combination of the following as its characterising components:
(a) a carnitine selected from the group comprising L-carnitine and propionyl L-carnitine or one of their pharmacologically acceptable salts or mixtures thereof, and
(b) a *Crataegus* extract
is extremely effective in the prevention and treatment of cardiac and vascular dysfunctions, particularly myocardial insufficiency, coronary disorders, peripheral vascular disorders, fatigue, cardiac rhythm disorders and nervous erethism, as a result of the potent synergistic effect exerted by its components.

It has also been found that component (a) may advantageously comprise a further "carnitine", selected from the group comprising acetyl L-carnitine, valeryl L-carnitine, isovaleryl L-carnitine and butyryl L-carnitine or their pharmacologically acceptable salts or mixtures thereof.

As regards component (b), the extract of *Crataegus* (hawthorn) may consist of an extract of the berries, flowers, shoots or leaves (or a mixture of these) of plants belonging to the various species of the genus *Crataegus* (*Rosaceae* family), such as *Crataegus oxycantha, Crataegus monogyna, Crataegus levigata, Crataegus pinnatifida* and *Crataegus sinaica.*

These *Crataegus* extracts comprise liquid alcoholic or hydroalcoholic extracts or tinctures, nebulised extracts or dry powders, or any extract of *Crataegus* obtained with the usual conventional procedures well known to experts in pharmaceutical technology and in extraction methods.

*Crataegus* extracts are suitably standardised with regard to their component contents, according to the indications of the various pharmacopoeias. They are titled at least 2.2% in flavonoids or 18.75% in oligomeric procyanidins or 1% in vitexin-2-rhamnoside.

### Experimental tests

The isolated rabbit heart test was performed according to the technique described by Langendorff (Langendorff O., Pflügers Arch. Ges. Physiol. Menschen und Tiere, 61, 291, 1985), maintaining the heart at a constant pressure of 70 cm H₂O with a Tyrode's solution. *Crataegus* extract alone, or L-carnitine or propionyl L-carnitine alone, or combinations of L-carnitine plus *Crataegus* extract, or propionyl L-carnitine plus *Crataegus* extract were administered in the infusion fluid. The effect of the carnitines alone or in combination with *Crataegus* on left ventricular pressure (LVP), coronary flow (CF), cardiac rhythm (CR) and cardiac contractile force (CCF) were then evaluated.

The results of these tests (Table 1) indicate that both the carnitines, and particularly propionyl L-carnitine, and *Crataegus* extract are capable of favourably influencing the various cardiac and vascular parameters assessed in rabbit isolated heart.

The injection of 1 mL of Tyrode's solution containing 10 mg of L-carnitine or propionyl L-carnitine in the infusion fluid caused an increase both in myocardial contractile force and in coronary flow. Equally efficacious was the effect of the injection of *Crataegus* extract (0.15 mL). A particularly significant effect, however, was achieved with the administration of L-carnitine and *Crataegus* extract in combination.

In this case, in fact, there was potentiation of the effects, demonstrating that the combination of carnitines and *Crataegus* extract exerts an unexpected and surprising synergistic and potentiating effect. The effect of the composition according to the invention on coronary flow in the rat was also assessed *in vivo*. In these tests, coronary spasm was induced in the rats by means of intravenous injection of 1 U/kg of pitressin.

The reduced myocardial oxygenation induced by the inadequate blood supply caused characteristic electrocardiographic changes to the T wave (asphyxial T wave).

In this test, one hour before injection of pitressin, the rats were orally administered L-carnitine alone (350 mg/kg), or propionyl L-carnitine alone (350 mg/kg), or *Crataegus* extract (100 mg/kg), or a combination of L-carnitine or propionyl L-carnitine plus *Crataegus.*

With these tests, too, an unexpected and surprising synergistic effect was observed between the protective activity of the carnitines and that of *Crataegus.*

**Table 1**

| | Percentage increase vs controls | | | |
|---|---|---|---|---|
| Treatment | CCF | CF | LVP | CR |
| L-carnitine | 20 | 27 | 25 | 16 |
| Propionyl L-carnitine | 30 | 60 | 25 | 20 |
| *Crataegus* extract | 35 | 80 | 30 | 30 |
| L-carnitine + *Crataegus* extract | 50 | 155 | 30 | 30 |
| Propionyl L-carnitine + *Crataegus* extract | 85 | 190 | 35 | 35 |

**Table 2**

| Treatment | Protected rats | Weakly protected rats | Unprotected rats |
|---|---|---|---|
| L-carnitine | 0 | 3 | 7 |
| Propionyl L-carnitine | 2 | 4 | 4 |
| *Crataegus* extract | 2 | 3 | 5 |
| L-carnitine + *Crataegus* extract | 3 | 5 | 2 |
| Propionyl L-carnitine + *Crataegus* extract | 8 | 2 | 0 |

Some illustrative, non-limiting examples of compositions according to the invention are reported hereinbelow:

| | | |
|---|---|---|
| 1) | L-carnitine | 500 mg |
| | *Crataegus* dry extract (title 2.2% in flavonoids) | 100 mg |
| | | |
| 2) | L-carnitine | 500 mg |
| | *Crataegus dry* extract (titled 2.2% in flavonoids) | 250 mg |
| | | |
| 3) | L-carnitine | 500 mg |
| | Hydroalcoholic *Crataegus* extract (titled 2.2% in flavonoids) | 1 ml |
| | | |
| 4) | Propionyl L-carnitine | 500 mg |
| | *Crataegus dry* extract (titled 2.2% in flavonoids) | 100 mg |
| | | |
| 5) | Propionyl L-carnitine | 500 mg |
| | Hydroalcoholic *Crataegus* extract (titled 2.2% in flavonoids) | 1 mL |
| | | |
| 6) | L-carnitine | 250 mg |
| | Propionyl L-carnitine | 250 mg |
| | *Crataegus* dry extract (titled 2.2% in flavonoids) | 250 mg |
| | | |
| 7) | L-carnitine | 100 mg |
| | Acetyl L-carnitine | 100 mg |
| | Propionyl L-carnitine | 100 mg |
| | *Crataegus dry* extract (titled 2.2% in flavonoids) | 100 mg |
| | | |
| 8) | L-carnitine | 50 mg |
| | Acetyl L-carnitine | 50 mg |
| | Propionyl L-carnitine | 50 mg |
| | Isovaleryl L-carnitine | 50 mg |
| | Butyryl L-carnitine | 50 mg |
| | *Crataegus dry* extract (titled 2.2% in flavonoids) | 150 mg |
| | | |
| 9) | L-carnitine | 100 mg |
| | Acetyl L-carnitine | 100 mg |
| | *Crataegus dry* extract (titled 2.2% in flavonoids) | 100 mg |
| | Taurine | 100 mg |
| | α-Ketoglutaric acid | 100 mg |
| | Coenzyme Q₁₀ | 20 mg |
| | Resveratrol | 2 mg |
| | | |
| 10) | L-carnitine | 100 mg |
| | Acetyl L-carnitine | 100 mg |
| | Propyonil L-carnitine | 100 mg |
| | *Crataegus dry* extract (titled 2.2% in flavonoids) | 100 mg |
| | *Allium sativum* extract | 50 mg |
| | *Ginkgo biloba* extract | 50 mg |

What is meant by pharmacologically acceptable salt of L-carnitine or alkanoyl L-carnitine is any salt of these with an acid that does not give rise to unwanted toxic or side effects. Such acids are well known to pharmacologists and to experts in pharmaceutical technology.

Non-limiting examples of such salts, are the following: chloride; bromide; iodide; aspartate, acid aspartate; citrate, acid citrate; tartrate; phosphate, acid phosphate; fumarate, acid fumarate; glycerol phosphate; glucose phosphate; lactate; maleate, acid maleate; orotate; oxalate, acid oxalate; sulphate, acid sulphate; trichloroacetate; trifluoroacetate and methane sulphonate.

A list of FDA-approved pharmacologically acceptable salts is given in Int. J. of Pharm. 33, 1986, 201-217; this publication is incorporated hereinbelow by reference.

## Claims

1. A combination composition which comprises:
(a) a "carnitine" selected from the group comprising L-carnitine and propionyl L-carnitine or their pharmacologically acceptable salts or mixtures thereof;
(b) an extract of *Crataegus.*

2. The composition of claim 1, wherein the ingredient (a) further comprises a "carnitine" selected from the group comprising acetyl L-carnitine, valeryl L-carnitine, isovaleryl L-carnitine and butyryl L-carnitine or their pharmacologically acceptable salts or mixtures thereof.

3. The composition of claim 1 or 2, wherein the extract of *Crataegus* is an extract of berries, flowers, sprouts, leaves or mixture therof of plants belonging to *Crataegus oxycantha, Crataegus monogyna, Crataegus levigata, Crataegus pinnatifida* and *Crataegus sinaica* species.

4. The composition of claim 3, wherein the extracts of *Crataegus* comprise liquid, alcoholic, hydroalcoholic extracts, tinctures, nebulized extracts or dry powders of said *Crataegus* species.

5. The composition of claim 3 or 4, wherein said *Crataegus* extracts contain at least 2,2% by weight of flavonoids or 18.75% by weight of oligomeric procianides or 1% by weight of vitexine-2-rhamnoside.

6. The composition of any of the preceding claims wherein the pharmacologically acceptable salt of L-carnitine or alkanoyl L-carnitine is selected from the group comprising: chloride; bromide; iodide; aspartate, acid aspartate; citrate, acid citrate; tartrate; phosphate, acid phosphate; fumarate, acid fumarate; glycerophosphate; glucose phosphate; lactate; maleate, acid maleate; orotate; oxalate; acid oxalate; sulphate, acid sulphate; trichloroacetate; trifluoroacetate and methane sulphonate.

7. The composition of any of the preceding claims, which further comprises vitamins, coenzymes, mineral substances, aminoacids, and antioxidants.

8. The composition of any of the preceding claims, orally administrable, in the form of a dietary supplement.

9. The composition of any of claims 1 to 7, orally, parenterally, rectally, sublingually or transdermally administrable, in the form of a medicament.

10. The dietary supplement of claim 8, for the prevention of cardiovascular disorders, myocardial insufficiency, coronary disorders, vascular peripheral disorders, fatigue, cardiac arrythmias and erethism.

11. The medicament of claim 9, for the therapeutic treatment of cardiovascular disorders, myocardial insufficiency, coronary disorders, vascular peripheral disorders, fatigue, cardiac arrythmias and erethism.

12. The dietary supplement of claim 10, in solid, semi-liquid or liquid form.

13. The medicament of claim 11, in solid, semi-liquid or liquid form.

14. The dietary supplement of claim 12, in the form of tablets, lozenges, pills, capsules , granulates or syrups.

15. The medicament of claim 13, in the form of tablets, lozenges, pills, capsules, granulates, syrups, vials or drops.

## Patentansprüche

1. Kombinationszusammensetzung, umfassend:
(a) ein "Carnitin", ausgewählt aus der Gruppe, umfassend L-Carnitin und Propionyl-L-carnitin oder deren pharmakologisch akzeptablen Salze oder Mischungen davon;
(b) einen Extrakt von Crataegus.

2. Zusammensetzung nach Anspruch 1, worin der Bestandteil (a) weiterhin ein "Carnitin" umfaßt, ausgewählt aus der Gruppe, umfassend Acetyl-L-carnitin, Valeryl-L-carnitin, Isovaleryl-L-carnitin und Butyryl-L-carnitin oder deren pharmakologisch akzeptablen Salze oder Mischungen davon.

3. Zusammensetzung nach Anspruch 1 oder 2, worin der Crataegus-Extrakt ein Extrakt von Beeren, Blumen, Schößlingen, Blättern oder Mischungen davon von Pflanzen ist, die zu Crataegus oxycantha-, Crataegus monogyna-, Crataegus levigata-, Crataegus pinnatifida- und Crataegus sinaica-Spezies gehören.

4. Zusammensetzung nach Anspruch 3, worin die Extrakte von Crataegus flüssige, alkoholische, hydroalkoholische Extrakte, Tinkturen, trübe Extrakte oder trockene Pulver dieser Crataegus-Spezies enthalten.

5. Zusammensetzung nach Anspruch 3 oder 4, worin die Crataegus-Extrakte zumindest 2,2 Gew.-% Flavonoide oder 18,75 % oligomere Procyanide oder 1 Gew.-% Vitexin-2-rhamnosid enthalten.

6. Zusammensetzung nach einem der vorhergehenden Ansprüche, worin das pharmakologisch akzeptable Salz von L-Carnitin oder Alkanoyl-L-carnitin ausgewählt ist aus der Gruppe, bestehend aus: Chlorid, Bromid, Iodid, Aspartat, saurem Aspartat, Citrat, saurem Citrat, Tartrat, Phosphat, saurem Phosphat, Fumarat, saurem Fumarat, Glycerylphosphat, Glucosephosphat, Lactat, Maleat, saurem Maleat, Orotat, Oxalat, saurem Oxalat, Sulfat, saurem Sulfat, Trichloracetat, Trifluoracetat und Methansulfonat.

7. Zusammensetzung nach einem der vorhergehenden Ansprüche, die weiterhin Vitamine, Co-Enzyme, Mineralsubstanzen, Aminosäuren und Antioxidantien umfaßt.

8. Zusammensetzung nach einem der vorhergehenden Ansprüche, die in der Form einer Diätergänzung oral verabreichbar ist.

9. Zusammensetzung nach einem der Ansprüche 1 bis 7, die in der Form eines Medikamentes oral, parenteral, rektal, sublingual oder transdermal verabreichbar ist.

10. Diätergänzung nach Anspruch 8, zur Verhinderung von kardiovaskulären Erkrankungen, Myokardinsuffizienz, Koronarerkrankungen, vaskulären peripheren Erkrankungen, Ermüdung, Herzarrhythmien und Erethismus.

11. Medikament nach Anspruch 9, zur therapeutischen Behandlung von kardiovaskulären Erkrankungen, Myokardinsuffizienz, Koronarerkrankungen, vaskulären peripheren Erkrankungen, Ermüdung, Herzarrhythmien und Erethismus.

12. Diätergänzungen nach Anspruch 10 in fester, halbflüssiger oder flüssiger Form.

13. Medikament nach Anspruch 11 in fester, halbflüssiger oder flüssiger Form.

14. Diätergänzung nach Anspruch 12, in der Form von Tabletten, Pastillen, Pillen, Kapseln, Granulaten oder Sirupen.

15. Medikament nach Anspruch 13, in der Form von Tabletten, Pastillen, Pillen, Kapseln, Granulaten, Sirupen, Ampullen oder Tropfen.

## Revendications

1. Composition qui comprend, en combinaison :
(a) une "carnitine" choisie parmi le groupe comprenant la L-carnitine et la propionyl-L-carnitine ou leurs sels pharmaceutiquement acceptables ou des mélanges de ceux-ci ;
(b) un extrait de *Cratoegus.*

2. Composition selon la revendication 1, dans laquelle l'ingrédient (a) comprend en outre une "carnitine" choisie parmi le groupe comprenant l'acétyl-L-carnitine, la valéryl-L-carnitine, l'isovaléryl-L-carnitine et la butyryl-L-carnitine ou leurs sels pharmaceutiquement acceptables ou des mélanges de ceux-ci.

3. Composition selon la revendication 1 ou 2, dans laquelle l'extrait de *Cratoegus* est un extrait de baies, de fleurs, de pousses, de feuilles ou un mélange de ceux-ci, de plantes appartenant aux espèces *Cratoegus oxycantha, Cratoegus monogyna, Cratoegus levigata, Cratoegus pinnatifida* et *Cratoegus sinaica*.

4. Composition selon la revendication 3, dans laquelle les extraits de *Cratoegus* comprennent des extraits liquides, alcooliques, hydroalcooliques, des teintures, des extraits nébulisés ou des poudres sèches desdites espèces de *Cratoegus.*

5. Composition selon la revendication 3 ou 4, dans laquelle les extraits de *Cratoegus* contiennent au moins 2,2% en poids de flavonoïdes ou 18,75% en poids de procianides oligomériques ou 1% en poids de vitexine-2-rhamnoside.

6. Composition selon l'une quelconque des revendications précédentes, dans laquelle le sel pharmaceutiquement acceptable de la L-carnitine ou de la L-carnitine alcanoylée est choisi parmi le groupe comprenant : le chlorure ; le bromure ; l'iodure ; l'aspartate, l'aspartate acide ; le citrate, le citrate acide ; le tartrate ; le phosphate, le phosphate acide ; le fumarate, le fumarate acide ; le glycérophosphate ; le phosphate de glucose ; le lactate ; le maléate, le maléate acide ; l'orotate ; l'oxalate acide ; le sulfate, le sulfate acide ; le trichloroacétate ; le trifluoroacétate et le sulfonate de méthane.

7. Composition selon l'une quelconque des revendications précédentes, qui comprend en outre des vitamines, des co-enzymes, des substances minérales, des acides aminés et des antioxydants.

8. Composition selon l'une quelconque des revendications précédentes, administrable par voie orale sous la forme d'un supplément diététique.

9. Composition selon l'une quelconque des revendications 1 à 7, administrable par voie orale, parentérale, rectale, sublinguale ou transdermique, sous la forme d'un médicament.

10. Supplément diététique selon la revendication 8, pour la prévention de maladies cardiovasculaires, d'une insuffisance du myocarde, de maladies coronariennes, de maladies périphériques vasculaires, de la fatigue, d'arythmies cardiaques et de l'éréthisme.

11. Médicament selon la revendication 9, destiné au traitement thérapeutique de maladies cardiovasculaires, d'une insuffisance du myocarde, de maladies coronariennes, de maladies périphériques vasculaires, de la fatigue, d'arythmies cardiaques et de l'éréthisme.

12. Supplément diététique selon la revendication 10, sous forme solide, semi-solide ou liquide.

13. Médicament selon la revendication 11, sous forme solide, semi-solide ou liquide.

14. Supplément diététique selon la revendication 12, sous forme de pilules, de tablettes, de comprimés, de gélules, de granulés ou de sirops.

15. Médicament selon la revendication 13, sous forme de pilules, de tablettes, de comprimés, de gélules, de granulés, de sirops, d'ampoules ou de gouttes.
